## Europäisches Patentamt
## European Patent Office
## Office européen des brevets

(19)

(11) Veröffentlichungsnummer: **0 276 330**
**A1**

# EUROPÄISCHE PATENTANMELDUNG

Veröffentlicht nach Art. 158 Abs. 3 EPÜ

(21) Anmeldenummer: **87905562.2**

(22) Anmeldetag: **31.07.87**

Daten der zugrundeliegenden internationalen Anmeldung:

(86) Internationale Anmeldenummer:
**PCT/SU87/00085**

(87) Internationale Veröffentlichungsnummer:
**WO88/00973 (11.02.88 88/04)**

(51) Int. Cl.³: **C 12 N 15/00**
**A 61 K 39/29**

FORTSETZUNG ERFINDER: PRIKHODKO; BLINOV;
SERPINSKY; URMANOV; KRAVCHENKO;
ANDZHAPARIDZE; CHERNOS; ANTONOVA.

(30) Priorität: **31.07.86 SU 4102079**

(43) Veröffentlichungstag der Anmeldung:
**03.08.88 Patentblatt 88/31**

(84) Benannte Vertragsstaaten:
**CH DE FR GB LI**

(71) Anmelder: **INSTITUT BIOORGANICHESKOI KHIMII
IMENI M.M. SHEMYAKINA AKADEMII NAUK SSR
ul. Miklukho-Maklaya, 16/10
Moscow, 117871(SU)**

(71) Anmelder: **INSTITUT POLIOMIELITA I VIRUSNYKH
ENTSEFALITOV AKAD. MED. NAUK SSSR
Leninsky raion
Moskovskaya obl., 142782, p/o Inst. poliomelita(SU)**

(71) Anmelder: **VSESOJUZNY
NAUCHNO-ISSLEDOVATELSKY INSTITUT
MOLEKULYARNOI BIOLOGII
pos. Koltsovo NPO 'Vektor'
Novosibirskaya obl. Novosibirsky raion, 633159(SU)**

(71) Anmelder: **MOSKOVSKY
NAUCHNO-ISSLEDOVATELSKY INSTITUT VIRUSNYKH
PREPARATOV
1 Dubrovskaya ul. 15
Moscow, 109088(US)**

(72) Erfinder: **OVCHINNIKOV, Jury Anatolievich
Zvenigorodskaya ul. 14a-11
Moscow, 121433(SU)**

(54) **REKOMBINANTE PLASMID-DNS, KODIEREND FÜR DIE SYNTHESE VON POLYPEPTIDSUBSTANZEN FÜR EINEN IMPFSTOFF GEGEN HEPATITIS-A.**

(57) Rekombination-Plasmid-DNS, die die Synthese von Polypeptidsubstanzen für die Vakzine gegen Hepatitis A kodieren, setzten sich aus einem Fragment des HAV-Genoms und einem Expressionsvektor zusammen.

Als Fragment des HAV-Genoms verwendet man: ein Fragment mit einer Länge von 389 Basenpaaren, das das Fragment des Proteins VP1 mit 4 bis 129 Aminosäuren kodiert; ein Fragment mit einer Länge von 3372 Basenpaaren, das alle strukturellen, beginnend mit 38 Aminosäuren des Proteins VP4, und nichtstrukturellen HAV-Proteine kodiert; ein Fragment mit einer Länge von 1243 Basenpaaren, das eine Polypeptidkette kodiert, die Fragmente der Proteine VP3 mit 110 bis 233 Aminosäuren und VP1 mit 1 bis 271 Aminosäuren aufweist; Fragmente mit einer Länge von 144 Basenpaaren, das ein Fragment des Proteins VP1 mit 4 bis 57 Aminosäuren kodiert; ein synthetisches DNS-Fragment, das ein Fragment des Proteins VP1 mit 11 bis 25 Aminosäuren kodiert, das folgende Struktur aufweist:

AATTCACAGTTAGTACTGAACAAAATGTACCAGATCCACAGGTAGGTA
TTGCGGTGTCAATCATGACTTGTTTTACATGGTCTAGGTGTCCATCCATA

oder ein Fragment mit einer Länge von 4296 Basenpaaren, das sich aus einem Fragment mit einer Länge von 3372 Basenpaaren zusammensetzt, das alle strukturellen und einige nichtstrukturelle HAV-Proteine kodiert, und aus einem Fragment mit einer Länge von 924 Basenpaaren, das die virale Protease kodiert. Als Expressionsvektor verwendet man Plasmid pUR 291, pUR 292, pIFN-fu 1, pIFN-fu 2 oder Plasmide pSPVV, das in den Vektor der Pockenvakzine LIVP eingebaut ist.

EP 0 276 330 A1

./...

(72) Erfinder: SVERDLOV, Evgeny Davidovich
ul. Matveevskaya 10-4-357
Moscow, 119517(SU)

(72) Erfinder: TSAREV, Sergei Anatolievich
Novoyasenevsky pr. 19-4-326
Moscow, 117593(SU)

(72) Erfinder: FROLOVA, Elena Ivanovna
ul. Alexya Tolstogo 24-19
Moscow, 103001(SU)

(72) Erfinder: ROKHLINA, Tatyana Oskarovna
ul. Osennaya, 2-9
Moscow, 121609(SU)

(72) Erfinder: ROSTAPSHOV, Viktor Mitrofanovich
ul. Volgina, 39-113
Moscow, 117437(SU)

(72) Erfinder: AZHIKINA, Tatyana Leodorovna
ul. Yartsevskaya, 24-1-35
Moscow, 121355(SU)

(72) Erfinder: ARSENIAN, Sergei Gagikovich
pr. Vernadskogo, 21-1-73
Moscow, 117331(SU)

(72) Erfinder: SNEZHKOV, Evgeny Valerievich
ul. Kirovogradskaya, 24-1-332
Moscow, 113519(SU)

(72) Erfinder: MARKOVA, Svetlana Vladimirovna
ul. Karbysheva, 4a-99
Krasnoyarsk, 660113(SU)

(72) Erfinder: KOVRIGA, Irina Evgenievna
ul. Volgina, 31-2-112
Moscow, 117437(SU)

(72) Erfinder: KUSOV, Jury Jurievich
ul. Tsurjupy, 7-2-77
Moscow, 117418(SU)

(72) Erfinder: NASTASHENKO, Tatyana Alexandrovna
p/o Institut poliomielita 6-67, Moskovskaya obl.
Leninsky raion, 142782(SU)

(72) Erfinder: VALYANO, Natalya Mikhailovna
ul. Ramenki, 7-1-41
Moscow, 117607(SU)

(72) Erfinder: BALAIAN, Mikhail Surenovich
Sokolnicheskaya naberezhnaya, 14/18-44
Moscow, 107113(SU)

(72) Erfinder: DROZDOV, Sergei Grigorievich
ul. Begovaya, 11-146
Moscow, 125284(SU)

(72) Erfinder: CHIZHIKOV, Vladimir Evgenievich
pos. Koltsovo, 10-44 Novosibirskaya obl.
Novosibirsky raion, 633159(SU)

(72) Erfinder: PETROV, Nikolai Alexeevich
pos. Koltsovo, 12-69 Novosibirskaya obl.
Novosibirsky raion, 633159(SU)

(72) Erfinder: VASILENKO, Stanislav Konstantinovich
pos. Koltsovo 1-210, Novosibirskaya obl.
Novosibirsky raion, 633159(SU)

(74) Vertreter: von Füner, Alexander, Dr. et al,
Patentanwälte v. Füner, Ebbinghaus, Finck Mariahilfplatz
2 & 3
D-8000 München 90(DE)

FIG. 1

REKOMBINATION-PLASMID-DNS, DIE DIE SYNTHESE VON POLYPEPTIDSUBSTANZEN FÜR DIE VAKZINE GEGEN HEPA- TITIS A KODIEREN

## Gebiet der Technik

Die vorliegende Erfindung bezieht sich auf Genen- **gineering** und auf Biotechnologie und insbesondere auf Rekombination-Plasmid-DNS, die die Synthese von Polypeptidsubstanzen für die Vakzine gegen Hepatitis A kodieren.

## Vorhergehender Stand der Technik

Hepatitis A ist umfassend eine verbreitete Erkrankung von Virusnatur. Ihr Erreger ist der Hepatitis-A- -Virus·(HAV) - RNS - der einen Virus aus der Familie Picornaviridae enthält.

Das Infizieren mit dem Virus erfolgt auf fäkal-oralem Wege, das Hauptprophylaxemittel gegen dieser Erkrankung sind die Sanitär- und Hygienemaßnahmen.

Abgesehen von allen eingeleiteten Maßnahmen wächst jedoch die Häufigkeit der Erkrankung an Hepatitis A in der ganzen Welt ständig an.

Ein effektives Verfahren zur Bekämpfung dieser Krankheit könnten Schutzimpfungen sein. Die Schwierigkeiten der Herstellung und der Reinigung des Hepatitis-A-Virus ermöglichten es jedoch bis jetzt noch nicht, weder eine kommerzielle virusintakte Totvakzine gegen Hepatitis-A, die auf der großangelegten Viruszüchtung mit seiner anschließenden Inaktivierung beruht, noch eine Genengineering-Vakzine, die auf der Klonierung eines Virusgenoms sowie auf der Expression seiner Einzelfragmente in verschiedenen Systemen beruht, zu entwickeln. Der letzte Weg erscheint jedoch gegenwärtig besonders aussichtsvoll.

Für die Herstellung der Genengineering-Vakzine ist es vor allem notwendig, das Genom des Hepatitis-A-Virus zu klonieren, seine Struktur und potentielle Abschnitte zu ermitteln, die die Antigendeterminanten kodieren. Als voraussichtliche Kandidaten für solche Abschnitte können Gene der Struktureiweißstoffe auftreten, die in der Zusammensetzung eines Virusteilchens enthalten sind.

Die ersten Arbeiten zur Klinierung und zur Ermittlung der primären Struktur der Gene der HAV-Kapsideiweißstoffe wurden 1985 veröffentlicht (J. Virol, v. 55, 1985, D.L. Linemeyer et al. "Molecular cloning and partial sequencing of hepatitis A viral cDNA", pp. 247-255; Proc. Natl. Acad. Sci. USA, v. 82, 1985 (April), Baroudy et al. "Sequence analysis of hepatitis A virus cDNA coding for capsid proteins and RNA polymerase", pp. 2143-2147; Proc. Natl. Acad. Sci. USA, v. 82, 1985 (May), Nararjn R. et.al. "Primary Structure and gene organization of human hepatitis A virus", pp.2627-2631; Bericht der Akademie der Wissenschaften der UdSSR, B. 285, Nr. 4, 1985, Ju.A. Ovchinnikov "Sequenz 3372 der Nukelotidkettenglieder RNS des Hepatitis-A-Virus, die die Kapsid-VP4-VP1 und einige nichtstrukturelle Proteine kodieren" "Posledovatelnost 3372 nukleotidnykh zveniev RNK virusa gepatita A, kodirujuschaya kapsidnye VP4 - VP1 i nekotorye nestrukturnye belki".

Gegenwärtig ist unbekannt, welcher Teil des HAV-Genoms die Antigendeterminanten kodiert, deshalb erfolgt ihr Nachweis empirisch.

Da es bekannt ist, daß eine Immunantwort oft nicht nur von der primären Struktur des jeweiligen Antigens, sondern auch von seiner räumlichen Organisation abhängig ist, gilt als ein wichtiger Moment bei der Entwicklung einer Genengineering-Vakzine nicht nur die Wahl eines Genomfragmentes, das die Antigendeterminante kodiert, sondern auch das System der Expression dieser Antigendeterminante. Dabei können verschiedene Systeme der Expression, die einunddasselbe HAV-Genom-Fragment enthalten, Substanzen produzieren, die sich durch ihre Eigenschaften prinzipiell unterscheiden.

Beschrieben wurden Rekombination-Plasmid-DNS (Plasmide), die die Synthese der Substanzen, die die Fragmente der HAV-Proteine aufweisen, in den bakteriellen Zellen für die Vakzine gegen Hepatitis A (EP, B, 154587) kodieren. In diesen Plasmiden wurden zwei Fragmente des HAV-Genoms (Stamm CR326) verwendet.

Eines davon kodiert das Fragment des Kapsid-Proteins VPI mit 57 bis 280 Aminosäuren und das andere kodiert Polypeptid, das Fragmente von zwei strukturellen Proteine VP3 (mit 100 bis 223 Aminosäuren) und VP1 (mit 1 bis 280 Aminosäuren) aufweist. Es wurde nachgewiesen, daß bei der Expression dieser Sequenzen im Vektor pCQV2 in den Zellen E.coli ein Produkt eintsteht, das Antigendeterminanten eines nativen Virus enthält. In dieser Arbeit fehlen jedoch Angaben, die die Fähigkeit der hergestellten Substanzen nachweisen, die Entstehung von Antikörpern bei der Einführung einem Menschen oder den Tieren hervorzurufen, was eine der Hauptanforderungen bei der Herstellung eines Genengineering-Vakzine darstellt.

Offenbarung der Erfindung

Der Erfindung liegt die Aufgabe zugrunde, neue Rekombination-Plasmid-DNS zu entwickeln, die die Synthese von Polypeptidsubstanzen für die Vakzine gegen Hepatitis A kodieren, die die Synthese in den bakteriellen und tierischen Zellen der Polypeptide bewirken, die fähig sind, die Entstehung von Antikörpern bei ihrer Einführung den Tieren beziehungsweise die Synthese solcher Polypeptide im Organismus von Menschen und Tieren zu induzieren.

Die angemeldeten Rekombination-Plasmid-DNS sind neu und wurden in der Literatur nicht beschrieben.

Die Aufgabe wurde dadurch gelöst, daß, erfindungsgemäß, die angemeldeten Rekombination-Plasmid-DNS, die die Synthese von Polypeptidsubstanzen der Vakzine gegen die Hepatitis A kodieren, aus einem Fragment des HAV-Genoms und einem Expressionsvektor bestehen, wobei man als Fragment des HAV-Genoms verwendet:

- Fragment mit einer Länge von 389 Basenpaaren, das das Protein-Fragment VP1 mit 4 bis 129 Aminosäuren kodiert;

- Fragment mit einer Länge von 3372 Basenpaaren, das alle strukturellen, beginnend mit 38 Aminosäuren des Proteins VP4, und nichtstrukturelle HAV-Proteine kodiert;

- Fragment mit einer Länge von 1243 Basenpaaren, das eine Polypeptidkette kodiert, die Fragmente der Proteine VP3 mit 110 bis 233 Aminosäuren und VP1 mit 1 bis 271 Aminosäuren enthält;

- Fragment mit einer Länge von 144 Basenpaaren, das ein Fragment des Proteins VP1 mit 4 bis 57 Aminosäuren kodiert;

- synthetisches DNS-Fragment, das ein Fragment des Proteins VP1 mit 11 bis 25 Aminosäuren kodiert, das folgende Struktur aufweist:

AATTCACAGTTAGTACTGAACAAAATGTACCAGATCCACAGGTAGGTATTGCG
    GTGTCAATCATGACTTGTTTTACATGGTCTAGGTGTCCATCCATA;

oder

- Fragment mit einer Länge von 4296 Basenpaaren, das aus einem Fragment mit einer Länge von 3372 Basenpaaren besteht, das alle strukturellen und einige nichtstrukturelle HAV-Proteine kodiert, und Fragment mit einer Länge von 924 Basenpaaren, das die Virusprotease kodiert; und man als Expressionsvektor verwendet:

- Plasmid pUR 291;
- Plasmid pUR 292;
- Plasmid pIFN-fu 1;
- Plasmid pIFN-fu 2 oder
- Plasmid pSPVV, das in den Vektor der Pockenvakzine LIVP eingebaut ist.

Die gewählten Fragmente des HAV-Genoms enthalten Sequenzen, die die potentiellen Antigendeterminanten des Virus kodieren. Als solche Fragmente können insbesondere Fragmente des HAV-Genoms des HAS-15-Stammes auftreten (J.Clinical v.Microb., v. 23, N 3, 1986, C.M. Wheeler et al. "Absorbtion, purification and growth charakteristics of hepatitis A virus strain Has-15, propagated in total Rhesus monkey kidney cells",pp.434--440), die vorher kliniert wurden (Bericht der Akademie der Wissenschaften der UdSSR, B. 285, Nr. 4, 1985, Ju.A. Ovchinnikov "Posledovatelnost 3372 nucleotidnykh zvenjev RNK virusa gepatita A, codirujuschaya kapsidnye VP4-VP1 i nekotorye nestrukturnye belki von 1014 - 1018" - Sequenz 3372 der Nukleotidketten RNS des Hepatitis-A-Virus, die die Kapsid- VP4-VP1 und einige nichtstrukturellen Proteine von 1014 bis 1018 kodiert).

Die gewählten Expressionsvektoren bewirken die Synthese der erforderlichen Virusproteine in bakteriellen beziehungsweise in tierischen Zellen.

Plasmide pUR 291, pUR 292 /EMBO J., v.2, No. 10, 1983, U.Rüther and B.Müller Hill "Easy identifikation of cDNA-clones", pp. 1791-1794/ bewirken die Synthese der Polypeptidsubstanzen in bakteriellen Zellen, beispielsweise in den Stämmen E.coli K 12. Die Polypeptidsubstanzen stellen die mit β-Galaktose verschmolzene Fragmente der HAV-Proteine dar.

Die Plasmide pIFN-fu 1 und pIFN-fu 2 setzen sich aus einem größeren EcoRI-PstI-Fragmenten des Plasmids pBR 322, einem Gen aus reifem Gammainterferon des Menschen zusammen, das sich unter der Kontrolle des trp--Promotors E.coli befindet, in dem Terminierungscodon mit Hilfe der Restriktionsendonuklease HinfI beseitigt ist und durch ein synthetische DNS-Fragmente ausgetauscht ist. Die Struktur dieser Fragmente ist wie folgt:
AGT CAG ATG CTG TTT CAA GGT CGA AGA GCA TCC CAG ACT GCA
    GTC TAC GAC AAA GTT CCA GCT TCT CGT AGG GTC TG
AGT CAG ATG CTG TTT CAA GGT CGA AGA GCA TCC CAA GAT C.TG CA
    GTC TAC GAC AAA GTT CCA GCT TCT CGT AGG GTT CTA G

Plasmide pIFN-fu 1 und pIFN-fu 2 weisen eine Länge von 4500 Basenpaaren auf, enthalten ein Gen der Resistenz gegenüber Tetracyclin und gewährleisten die Synthese von Substanzen für Vakzinen in bakteriellen Zellen, beispielsweise in den Stämmen E.coli K 12, die die mit dem Gamma-Interferon des Menschen zusammengeschmolzenen HAV-Proteine darstellen.

Wie oben erwähnt wurde, kann man als Expressionsvektor das Plasmid pSPVV anwenden, das in den Vektor der Pockenvakzine eingebaut wird. Das Plasmid pSPVV ist ein Derivat des Plasmids pSP 64 (Nucleic Acids Res., v. 12, No. 18, 1984. D.A. Melton et al., Efficient in

- 6 -

vitro synthesis of biologically active RNA and RNA hybridization probes from plasmids containing a bacteriophage SP6 promoter", pp. 7035-7056).

Das Plasmid pSPVV weist eine Länge von 5800 Basenpaaren, enthält ein Gen der Resistenz gegenüber Ampizillin, ein Fragment des frühzeitigen Pockenpromotors, tk-Gens, und eine Polylinkersequenz. Dieser Expressionsvektor gewährleistet die Synthese von Polypeptidsubstanzen sowohl in tierischen Zellen als auch in Organismen von Tieren. In diesem Fall werden die Polypeptidsubstanzen nur in Form von Virussequenzen ohne andere Proteine synthetisiert.

Manchmal wurden Adaptorfragmente von DNS, die eine geringe Anzahl von Aminosäuren kodieren, für die Gewährleistung des erforderlichen Rahmens für die Ablesung der Virussequenzen beim Einbau in die Vektoren verwendet.

Auf der Grundlage der vorgeschlagenen Rekombination-DNS wurden Polypeptidsubstanzen für die Vakzine gegen HAV erhalten. Die Polypeptidsubstanzen werden den Meerschweinchen und den Kaninchen eingeführt, in einigen Wochen nach dem Abschluß der Immunisierung ermittelt man den Gehalt an Antikörpern in ihrem Blut, die fähig sind, infektiöse HAV zu binden.

Die Immunisierung wird wie folgt durchgeführt.

Mit den hergestellten Polypeptidsubstanzen immunisiert man Männchen der Meerschweinchen mit einem Gewicht von 180 bis 200 g, berechnet je 10 μg Substanz je eine Injektion, bei der Immunisierung mit Produkten, die sich im Polyakrylamidgel befinden, beziehungsweise 20 μg je eine Injektion bei der Immunisierung mit einer Lösung. Vor Beginn der Immunisierung entnimmt man das Blut bei den Tieren und prüft man auf "Unspezifik". Bei der Arbeit verwendet man nur Meerschweinchen, die keine unspezifischen Reaktionen gegenüber HAV zeigen. Die Immunisierungsbehandlungen betragen 0, 14, 28, 42 und manchmal 72 Tage. Das Blut für die Herstellung des zu untersuchenden Serums nimmt man aus dem Herzen am 14. Tag nach der 4. oder nach der 5. Immunisierung.

Bei der Immunisierung von Kaninchen, deren Serum keine unspezifischen Reaktionen gegenüber HAV zeigt, werden die Präparate in einer Menge von 100 μg je eine Injektion den Männchen der Kaninchen mit einem Gewicht von 1,5 bis 2 kg eingeführt. Insgesamt werden 5 Injektionen mit einem zweiwöchigen Interval durchgeführt das Blut entnimmt man nach der 4. oder nach der 5. Immunisierung aus der Ohrvene.

Die Polypeptidsubstanzen werden den Kaninchen an mehreren Punkten eingeführt: intramuskulär und subkutan, manchmal in die hintere Pfote. Den Meerschweinchen: intramuskulär, intraperitoneal und subkutan.

Bei der ersten Injektion verwendet man das komplette Freud-Adjuvant und bei den nächsten Injektionen das unkomplette Adjuvant. Die Präparate werden mit dem Adjuvant unmittelbar vor der Einführung sorgfältig homogenisiert.

Der Gehalt an virusspezifischen Antikörpern im Serum der immunisierten Tiere ermittelt man wie folgt. Chlorvinyl-Immunpanels sensibilisiert man mit dem Anti--HAV-Serum der Rekovaleszente innerhalb von 16 bis 18 Stunden bei Raumtemperatur. Die Panels werden dreimal mit e i n e r Pufferlösung v o n 0,01 PhBL bei einem pH-Wert von 7,46 gewachen, die 0,05% Tween-20 enthält. Dann setzt man in die Alveolen virales HAV-Antigen zu, das aus der Leber der experimentell infizierten Affen ausgeschieden wird oder in der Kultur der Nierenzellen des Embryons von Macacus rhesus vermehrt wurde, und inkubiert man bei Raumtemperatur innerhalb von 16 bis 18 Stunden. Nach dem Waschen der Panels bringt man das Peroxydasekonjugat des Anti-HAV-Immunoglobulins der Klasse G ein, inkubiert man während 2 Stunden bei 37°C, wäscht man und entwickelt man wie üblich mit Orthopehnylendiamin. Als Serumtiter gilt die Verdünnung des letzteren, die eine 50%ige Blockierung des Antigens gegenüber der Reaktion bewirkt, die in Gegenwart eines normalen /nichtblockierten/ Serums oder überhaupt ohne Serum durchgeführt wird. Die Kontrolle der Reaktion erfolgt mit Hilfe eines Spektrophotometers.

Die Expressionsprodukte, die die Entstehung von Antikörpern hervorrufen, die fähig sind, mit den humanen Antikörpern für die Bindung mit HAV bei einer Verdünnung nicht unter 1:20 zu konkurieren, werden als perspektivische Polypeptide für die Herstellung der Genengineering-Vakzine betrachtet.

Die erfindungsgemäßen Rekombination-Plasmid-DNS gewährleisten die Synthese in bakteriellen beziehungsweise in tierischen Zellen der Polypeptidsubstanzen, die fähig sind, die Entstehung von Antikörpern gegenüber den Hepatitis-A-Virus sowie die Synthese dieser Substanzen im Organismus der Tiere und des Menschen zu induzieren. Die erfindungsgemäßen Rekombination-Plasmid-DNS auf der Grundlage der Pockenvakzine LIVP kann man bei der unmittelbaren Vakzination, beispielsweise, von Kaninchen verwenden. In diesem Fall erfolgt die Expression von Polypeptidsubstanzen unmittelbar im Organismus von Tieren.

Die Erfindung wird an Hand der Beispiele erläutert und mit folgenden Figuren der graphischen Darstellungen veranschaulicht:

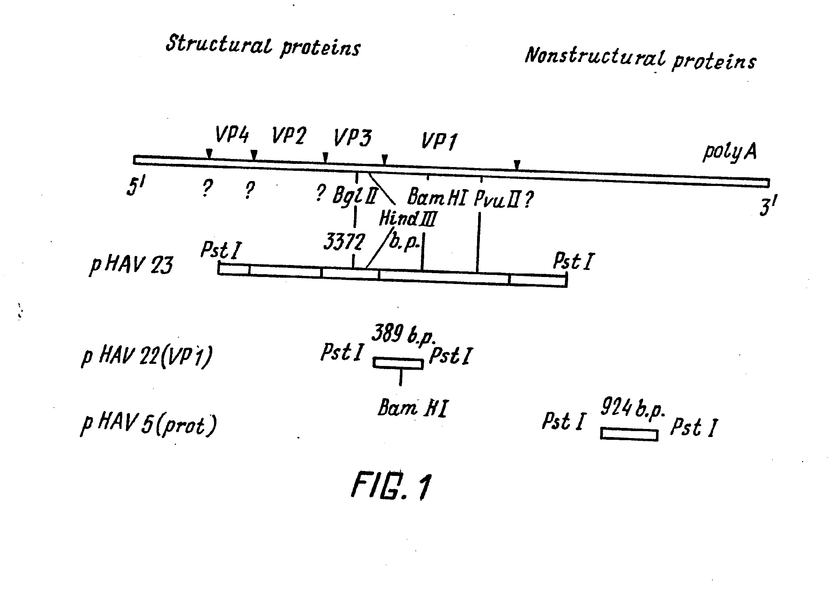

Fig. 1 - Aufbauschema eines HAV-Genoms und seiner klonierten Fragmente;

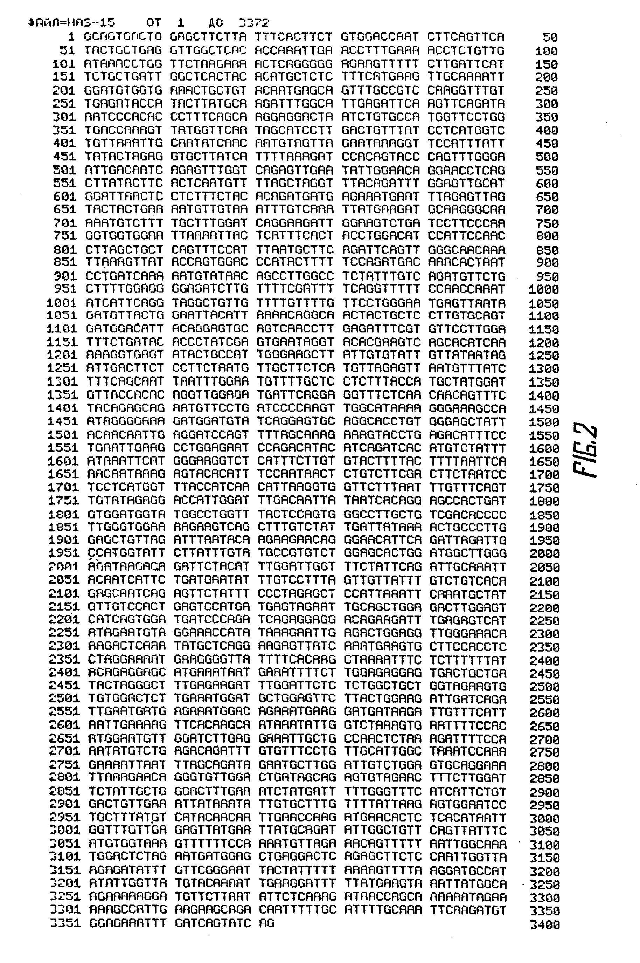

Fig. 2 - primäre Struktur eines Fragmentes des HAV-Genoms /Stamm HAS-15/ mit einer Länge von 3372 Basenpaaren, das alle strukturellen /beginnend mit 38 Aminosäuren des Proteins VP4) und einen Teil der nichtstrukturellen HAV-Proteine kodiert;

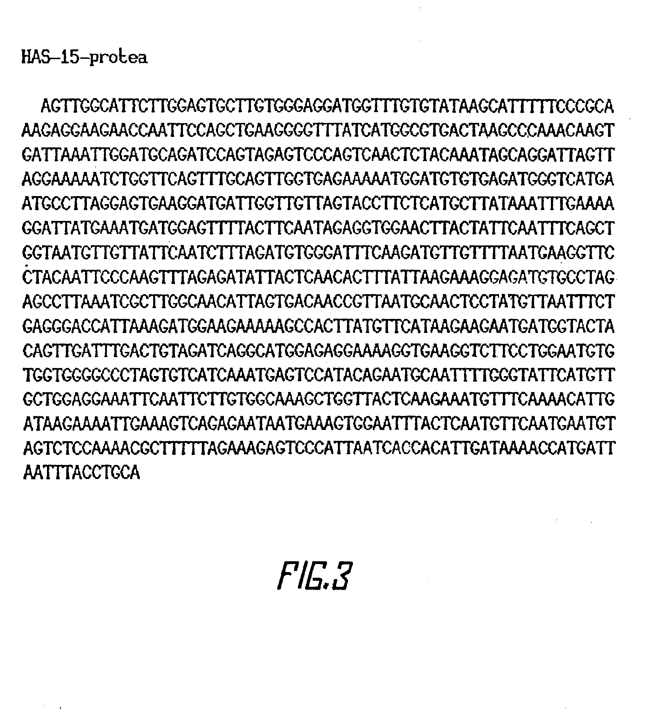

Fig. 3 - primäre Struktur eines Fragmentes des HAV-Genoms /Stamm HAS-15/ mit einer Länge von 924 Basenpaaren, das virale Protease kodiert;

Fig. 4 - physikalische Karte des Expressionsplasmids pUR 291-HAV 22;

Fig. 5 - physikalische Karte des Expressionsplasmids pUR 292-HAV 23;

Fig. 6 - physikalische Karte des Expressionsplasmids pIFN fu 1-HAV 22;

Fig. 7 - physikalische Karte des Expressionsplasmids pIFN-fu 1-VP3, I;

Fig. 8 - physikalische Karte des Expressionsplasmids pIFN-fu 2-HAV 23;

Fig. 9 - physikalische Karte des Expressionsplasmids pIFN-fu 1-VP1;

Fig. 10 - physikalische Karte des Expressionsplasmids pIFN-fu 1-VP1 (11-25);

Fig. 11 - physikalische Karte des Expressionsplasmids pSPVV-HAV-D;

Fig. 12 - physikalische Karte des Expressionsplasmids pSPVV-HAV-D-protea.

Das Verfahren zur Herstellung von Rekombination-Plamid-DNS beruht auf bekannten Methoden des Genengineering und besteht in dem Einbau von Fragmenten des HAV-Genoms in einen Expressionsvektor.

Beste Variante der Offenbarung der Erfindung

Zur besseren Erläuterung der vorliegenden Erfindung werden nachtehende konkrete Beispiele angeführt.

Beispiel 1. Rekombination-Plasmid-DNS pUR 291--HAV-22.

5 μg Plasmids pUR 291 spaltet man unter Standardbedingungen mit Restriktionsendonuklease Pst I. Hierfür inkubiert man ein Reaktionsgemisch, das 10 mMol tris-HCl, 10 mMol $MgCl_2$, 10 mMol 2-Merkaptoäthanol, 5 μg des DNS-Plasmids und 5 Aktivitätseinheiten des Ferments aufweist, bei einer Temperatur von $37^0C$ innerhalb von einer Stunde. Nach der Inkubation wird das Gemisch der Elektrophorese in einer niedrigschmelzenden Agarose ausgesetzt. Lineare Form des Plasmids p UR 291 scheidet man durch Phenolextraktion aus. Das hergestellte Fragment ligiert man mit einem Fragment Pst I mit einer Länge von 389 Basenpaaren, das aus dem Plasmid pHAV 22 ausgeschieden wurde. Mit einem Ligasegemisch transformiert man kompetente Zellen des Stammes E.coli BMH 71-18 und in den Klonen, die ein Plasmid mit einem eingebauten Fragment enthalten, ermittelt man die Orientierung des letzteren mit Hilfe der Restriktionsendonuklease BamHI.

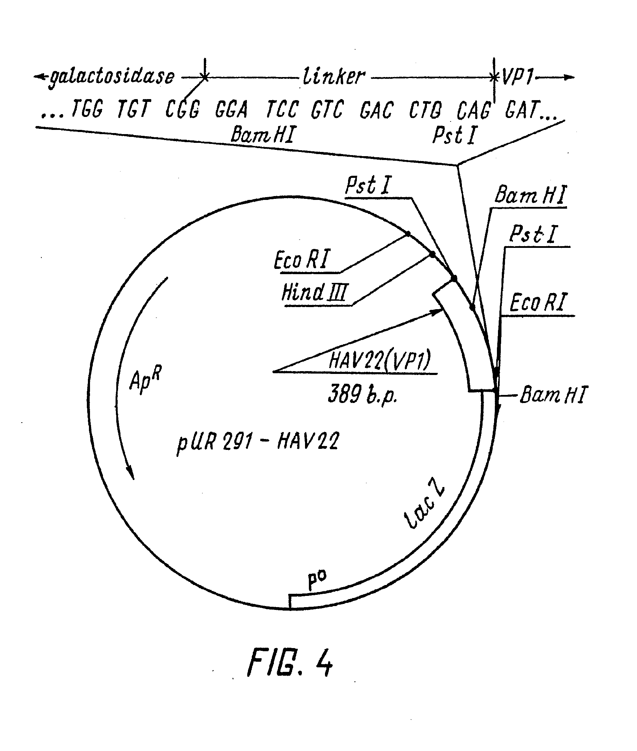

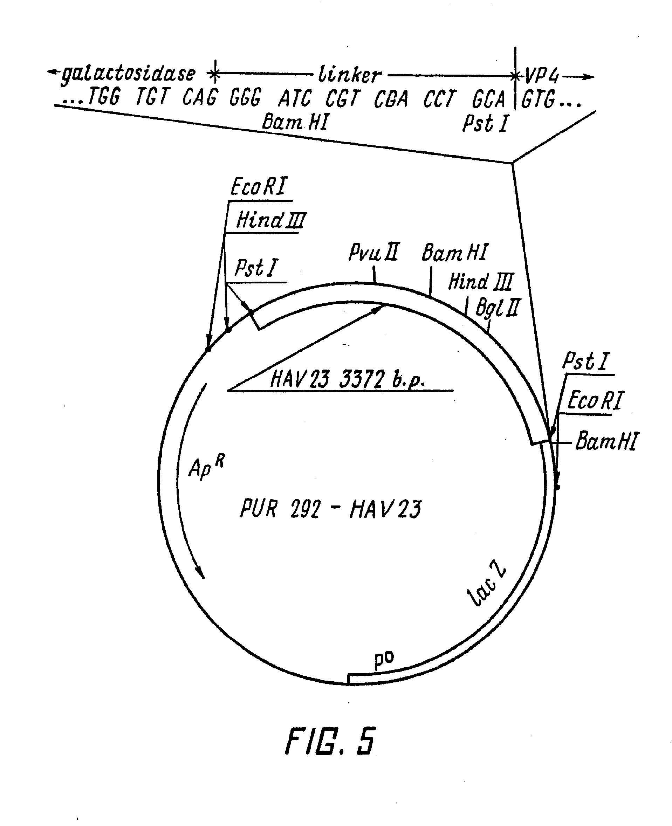

Klone, die eine Rekombination-Plasmid-DNS mit der erforderlichen Orientierung des eingebauten Fragmentes des HAV-Genoms (pUR 291-HAV 22, Fig. 4) aufweisen, werden im LB-Medium mit Ampizillin und IPTG angegliedert. Das synthetisierbare Hybridprotein, das komplette Beta-Galaktosidase und ein Fragment des Proteins VP1 HAV (mit 4 bis 129 Aminosäuren) aufweist, scheidet man aus bakteriellen Zellen aus und verwendet man für die Immunisierung von Meerschweinchen und Kaninchen.

In einigen Wochen nach dem Abschluß der Immunisierung testiert man das Serum der Meerschweinchen und Kaninchen auf Gehalt an Antikörpern, die fähig sind, sich mit dem nativen HAV in einer Konkurrenzreaktion mit humanen Antikörpern zu verknüpfen.

Infolge der Immunisierung wurden bei einem von den 3 Meerschweinchen und bei einem von den 3 Kaninchen im Serum Antikörper nachgewiesen, die fähig sind, sich mit nativem HAV zu verknüpfen.

Beispiel 2. Rekombination-Plasmid-DNS pUR 292--HAV 23.

5 μg Plasmids pUR 292 spaltet man unter Normalbedingungen mit der Restriktionsendonuklease Pst I. Hierfür wird das Reaktionsgemisch, das 10 mMol tris-HCl, 10 mMol MgCl$_2$, 10 mMol 2-Merkaptoäthanol, 5 μg DNS-Plasmid und 5 Aktivitätseinheiten des Fermentes aufweist, bei einer Temperatur von 37°C innerhalb von 1 Stunde inkubiert. Das hergestellte Fragment ligiert man mit einem Fragment Pst I mit einer Länge von 3372 Basenpaaren, das aus dem Plasmid pHAV 23 ausgeschieden wird. Nach der Inkubation setzt man das Reaktionsgemisch der Elektrophorese in der niedrigschmelzenden Agarose aus. Lineare Form des Plasmids pUR 292 extrahiert man durch Phenolextraktion.

Das hergestellte Ferment ligiert man mit Pst I mit einem Fragment mit einer Länge von 3372 Basenpaaren, das aus dem Plasmid pHAV 23 im Reaktionsgemisch ausgeschieden wurde, das 0,1 $\mu$g Vektor-DNS, 0,1 $\mu$g DNS-Einsatz in Gegenwart von rATP (0,1 mMol) aufweist. Mit dem Ligasegemisch transformiert man kompetente Zellen des Stammes E.coli BMH 71-18 und in den Klonen, die das Plasmid mit dem eingebauten Fragment enthalten, ermittelt man die Orientierung des letzteren mit Hilfe der Restriktionsendonuklease BamHI.

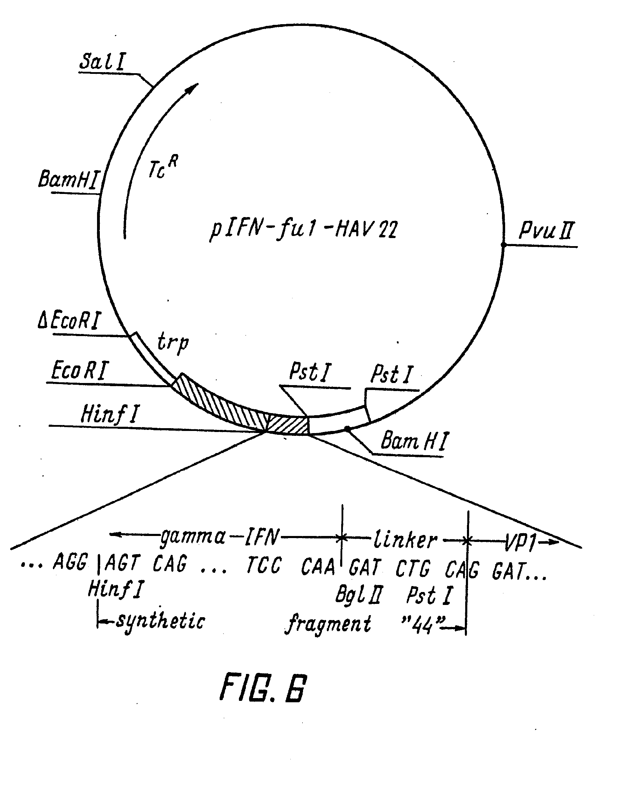

Die Klone, die eine Rekombination-Plasmid-DNS mit einer erforderlichen Orientierung des eingebauten Fragmentes des HAV-Genoms (pUR 292-HAV 23, Fig. 5) enthalten, gliedert man im LB-Medium mit Ampizillin und IPTC an. Das synthetisierbare Hybridprotein, das komplette Beta-Galaktose, alle strukturellen /beginnend mit 38 Aminosäuren des Proteins VP4) und einen Teil der nichtstrukturellen HAV-Proteine aufweist, extrahiert man aus bekteriellen Zellen und verwendet man für die Immunisierung von Meerschweinchen.

In einigen Wochen nach dem Abschluß der Immunisierung wird das Serum der Meerschweinchen auf Gehalt an Antikörpern testiert, die fähig sind, sich mit nativem HAV in der Konkurrenzreaktion mit humanen Antikörpern zu verknüpfen.

Im Ergebnis der Immunisierung wurden bei einem von den 4 Meerschweinchen in ihrem Serum Antikörper nachgewiesen, die fähig waren, sich mit nativem HAV zu verknüpfen.

Beispiel 3. Rekombination-Plasmid-DNS pIFN-fu 1-HAV 22.

5 $\mu$g Plasmids p IFN-fu 1 spaltet man unter Normalbedingungen mit der Restriktionsendonuklease Pst I, wie in Beispiel beschrieben. Das hergestellte Fragment ligiert man mit einem Fragment Pst I mit einer Länge von 389 Basenpaaren, das aus dem Plasmid pHAV 22 ausgeschieden wurde. Mit dem Ligasegemisch transformiert man kompetente Zellen des Stammes E.coli HB 101 und in den

Klonen, die das Plasmid mit dem eingebauten Fragment aufweisen, ermittelt man die Orientierung des letzteren mit Hilfe der Restriktionsendonuklease BglII und BamHI.

Die Klone, die eine Rekombination-Plasmid-DNS mit einer erforderlichen Orientierung des eingebauten Fragmentes des HAV-Genoms (pIFN-fu-1-HAV 22  Fig. 6)  enthalten, gleidert man im M-9-Medium mit Tetrazyklin und mit der Beta-Indolylakrylsäure an. Das synthetisierbare Hybridprotein, das komplettes Gamma-Interferon des Menschen und ein Fragment des Proteins VPI HAV /mit 4 bis 129 Aminosäuren/ aufweist, extrahiert man aus bakteriellen Zellen und verwendet man für die Immunisierung von Meerschweinchen und Kaninchen.

In einigen Wochen nach dem Abschluß der Immunisierung testet man das Serum der Meerschweinchen und Kaninchen auf Gehalt an Antikörpern, die fähig sind, mit nativem HAV in einer Konkurrenzreaktion mit humanen Antikörpern sich zu verknüpfen.

Mehrere der immunisierten Meerschweinchen und Kaninchen enthielten Antikörper in recht hohen Konzentration, die fähig waren, sich mit nativem Virus zu verknüpfen.

Beispiel 4. Rekombination-Plasmid-DNS pIFN-fu 1-VP3, 1.

5 $\mu$g Plasmids pIFN-fu-1-HAV 22 spaltet man unter den Normalbedingungen mit der Restriktionsendonuklease BglII und dann Pst I, wie in Beispiel 1 beschrieben. Das  hergestellte größere Fragment ligiert man mit BglII-(Pvv-II)Pst I-Fragment mit einer Länge von 1243 Basenpaaren, das aus dem Plasmid pHAV 23 ausgesdondert ist. Mit dem Ligasegemisch transformiert man kompetente Zellen des Stammes E.coli HB 101 und in den Klonen, die ein Plasmid mit einem eingebauten Fragment des HAV-Genoms aufweisen, ermittelt man die Orientierung des letzteren mit Hilfe der Restriktionsendonuklease EcoRI und BamHI.

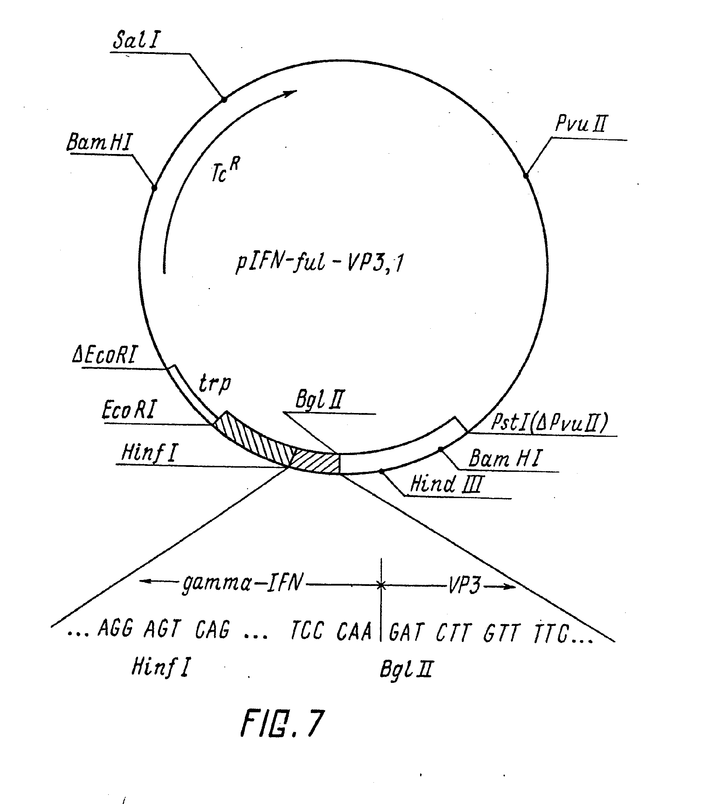

Die Klone, die eine Rekombination-Plasmid-DNS mit einer erforderlichen Orientierung des Fragmentes (pIFN--fu 1-VP3, 1, Fig. 7) enthalten, gliedert man im M-9-Medium mit Tetrazyklin und mit der Beta-Indolylakrylsäure - an. Das synthetisierbare Hybridprotein, das komplettes Gamma-Interferon des Menschen und Fragmente der Proteine VP3 (von 110 bis 233 Aminosäuren) und VPI (von 1 bis 271 Aminosäuren) des HAV aufweisen, extrahiert man aus bekteriellen Zellen und verwendet man für die Immunisierung von Meerschweinchen und Kaninchen.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Die Mehrheit der immunisierten Meerschweinchen und Kaninchen enthielten Antikörper in ausreichend hohen Konzentrationen, die fähig waren, sich mit nativem Virus zu verknüpfen.

Beispiel 5. Rekombination-Plasmid-DNS pIFN-fu 2-HAV 23.

5 $\mu$g Plasmids pIFN-fu2 spaltet man unter den Normalbedingungen mit der Restriktionsendonuklease PstI, wie in Beispiel 1 beschrieben. Das hergestellte Fragment ligiert man mit einem Fragment PstI mit einer Länge von 3371 Basenpaaren, das aus dem Plasmid pHAV 23 ausgeschieden ist. Mit dem Ligasegemisch transformiert man kompetente Zellen des Stammes E.coli HB 101 und in den Klonen, die ein Plasmid mit dem eingebauten Fragment des HAV-Genoms aufweisen, ermittelt man die Orientierung des letzteren mit Hilfe der Restriktionsendonukleasen BglII und EcoRI.

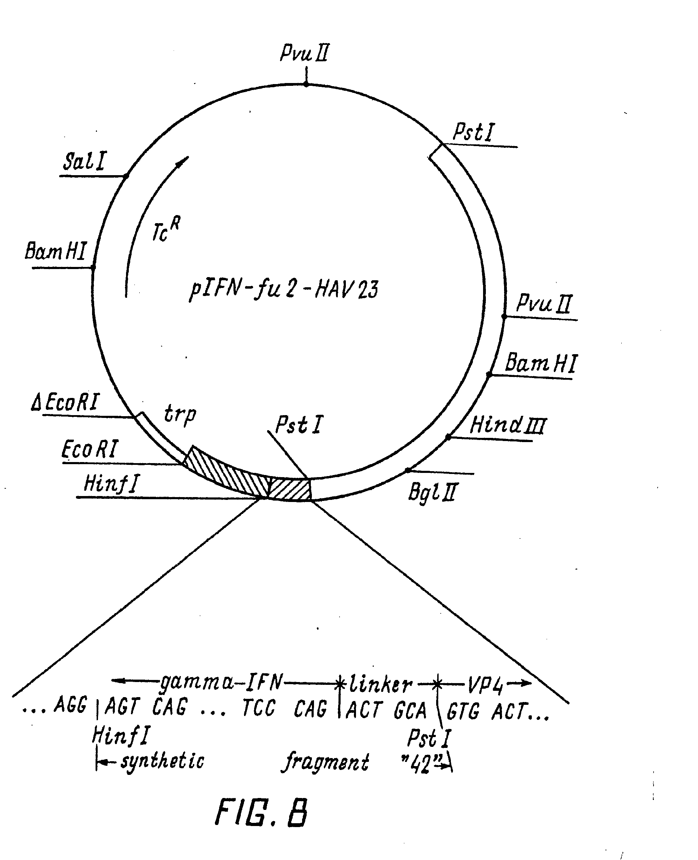

Die Klone, die Rekombination-Plasmid-DNS (pIFN-fu2-HAV 23, Fig. 8) mit einem Fragment in einer erforderlichen Orientierung aufweisen, gliedert man im M-9--Medium mit Tetrazyklin und mit Beta-Indolylakrylsäure an. Das synthetisierbare Hybridprotein, das das komplette Gamma-Interferon des Menschen und alle strukturellen /beginnend mit 38 Aminosäuren VP4/ und einen Teil der nichtstrukturellen HAV-Proteine aufweist, extrahiert man aus bakteriellen Zellen und verwendet man für die Immunisierung von Meerschweinchen und Kaninchen.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Die Mehrheit der immunisierten Meerschweinchen und Kaninchen enthielten Antikörper in ausreichend hohen Konzentrationen, die fähig waren, sich mit nativem Virus zu verknüpfen.

Beispiel 6. Rekombination-Plasmid-DNS pIFN-fu 1-VP1.

5 µg Plasmids pIFN-fu 1 spaltet man unter den Normalbedingungen mit der Restriktionsendonuklease Pst I, wie in Beispiel 1 beschrieben. Das hergestellte Fragment ligiert man mit Pst I-BamHI-Fragment mit einer Länge von 144 Basenpaaren, das ein Fragment des VPI-Proteins von 4 bis 57 Aminosäuren kodiert, das aus dem Plasmid pHAV 22 ausgeschieden ist, und mit einem synthetischen Adaptor, der folgende Struktur aufweist:

```
GAT CAA GTT TAA CTG CA
   GT CAA ATT G
```

Mit dem Ligasegemisch transformiert man kompetente Zellen des Stammes E. coli HB 101 und in den Klonen, die ein Plasmid mit dem eingebauten Fragment des HAV-Genoms aufweisen, ermittelt man die Orientierung des letzteren mit Hilfe der Restriktionsendonukleasen EcoRI und BamHI.

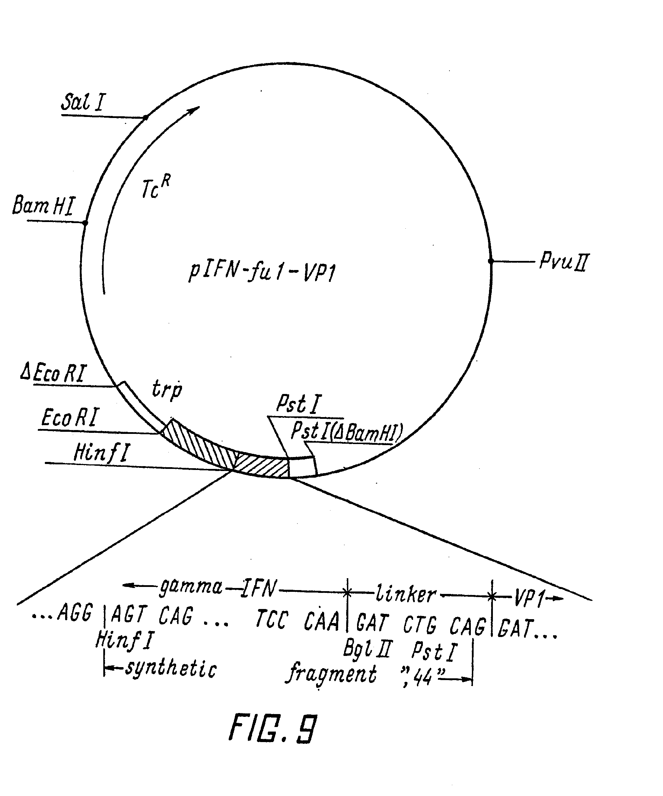

Die Klone, die Rekombination-Plasmid-DNS mit einer erforderlichen Orientierung des Fragmentes (pIFN-fu 1-VP1, Fig. 9) aufweisen, gliedert man im M-9-Medium mit Tetrazyklin und mit Beta-Indolylakrylsäure an. Das synthetisierbare Hybridprotein, das das komplette Gamma-Interferon des Menschen und ein Fragment VPI des HAV-Proteins /von 4 bis 57 Aminosäuren/ aufweist, extrahiert man aus bekteriellen Zellen und verwendet man für die

- 15 -

Immunisierung von Meerschweinchen und Kaninchen.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Die Mehrheit der immunisierten Meerschweinchen und Kaninchen enthielten Antikörper in ausreichend hohen Konzentrationen, die fähig waren, sich mit nativem Virus zu verknüpfen.

Beispiel 7. Rekombination-Plasmid-DNS pIFN-fu 1-VP1 (11-25).

5 $\mu$g Plasmids pIFN-fu 1 spaltet man unter Normalbedingungen mit der Restriktionsendonuklease PstI, wie in Beispiel 1 beschrieben. Das hergestellte Fragment ligiert man mit einem synthetischen DNS-Fragment, das ein Fragment des Proteins VP1 mit 11 bis 25 Aminosäuren kodiert, und mit einem Adaptorfragment, das folgende Struktur aufweist:

G GGT TCC G
AC GCT CCA AGG CTT AA

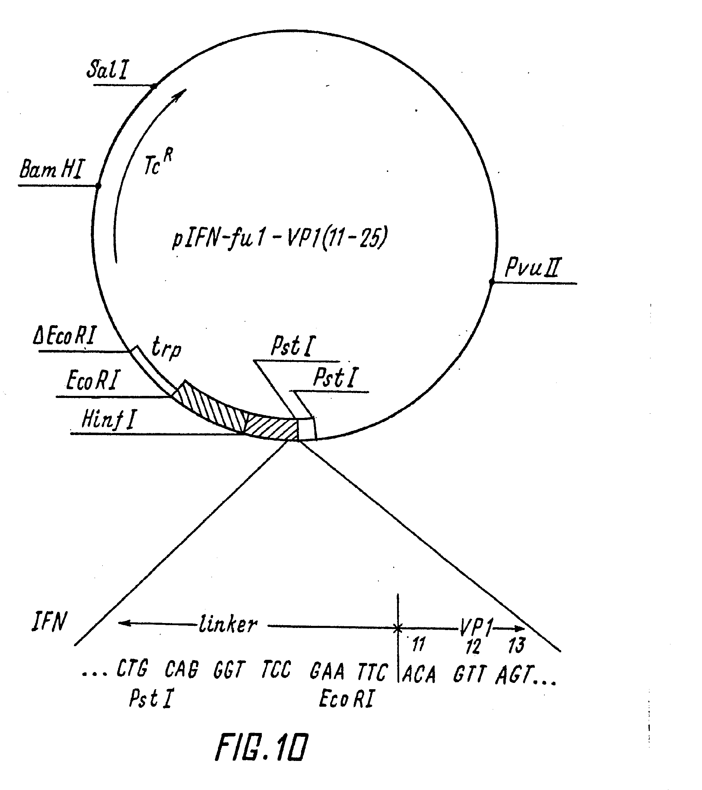

Die Klone, die Rekombination-Plasmid-DNS mit einem Fragment des HAV-Genoms in einer erforderlichen Orientierung (Plasmid pIFN-fu 1-VPI (11-25), Fig. 10) aufweisen, gliedert man im M-9-Medium mit Tetrazyklin und Beta-Indolylakrylsäure an. Das synthetisierbare Hybridprotein, das das komplette Gamma-Interferon des Menschen und ein Fragment des Proteins VPI HAV mit 11 bis 25 Aminosäuren aufweist, extrahiert man aus bakteriellen Zellen und verwendet man für die Immunisierung von Meerschweinchen und Kaninchen.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Einige der immunisierten Meerschweinchen und Kaninchen enthielten Antikörper in ausreichend hohen Konzentrationen, die fähig waren, sich mit nativem Virus zu verknüpfen.

Beispiel 8. Rekombination-Plasmid-DNS pSPVV-HAV-D.

5 μg Plamids pSPVV spaltet man unter den Normalbedingungen mit der Restriktionsendonuklease BamHI, analog
dem Beispiel 1. Das hergestellte Fragment ligiert man
mit PstI-Fragment des HAV-Genoms mit einer Länge von
3372 Basenpaaren, das aus dem Plasmid pHAV 23 ausgeschieden ist, und mit einem synthetischen HAV-Fragment folgender Struktur:

GAT CCT ATG AGG ACT GCA
GA TAC TCC TG

Mit dem Ligasegemisch transformiert man kompetente
Zellen des Stammes E.coli DHI und in den Klonen, die ein
Plasmid mit eingebautem Fragment enthalten, ermittelt
man die Orientierung des letzteren mit Hilfe der Restriktionsendonukleasen BamHI, PVUII, Hind III.

In der Rekombination-Plasmid-DNS, die ein Fragment
des HAV-Genoms in einer erforderlichen Orientierung aufweist, ermittelt man die Struktur der Angliederung im
Maxam-Gilbert-Verfahren.

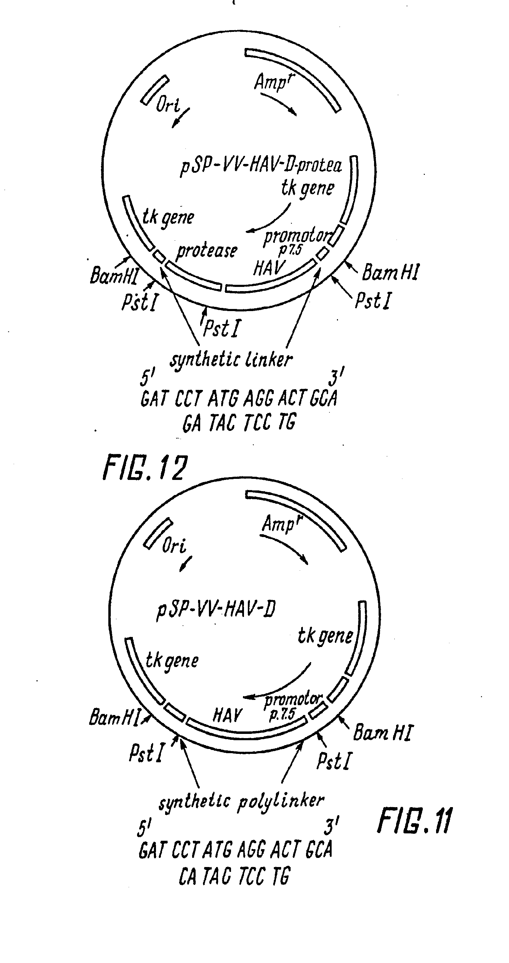

Die DNS-Plasmide pSPVV-HAV-D, Fig. 11 mit bestätigter Fusionsstruktur kodieren die Synthese eines Polypeptids, das alle strukturellen /beginnend mit 38 Aminosäuren des VP4-Proteins/ und einige nichtstrukturellen HAV-
-Proteine aufweist. Diese Plasmide verwendet man für
Transformation von Nierenzellen der grünen Meerkatze der
Linie CV1, die mit dem Virus der Pockenvakzine des Stammes LIVP infiziert wurden. Die Viruskultur wird nach
der Transformation auf embryonale Fibroblaste einer Ratte der überimpften Linie RAT2 TK-Phenotyp geimpft /Virology, v. 113, No. 1, 1981, W.C. Topp "Normal Rat Cell
Lines Deficient by Nuclear Thymidine Kinase", pp.408-411).

In Gegenwart des Bromdesoxyuridins kloniert man
Rekombinationsviren. Die Klone des Pockenvakzin-Virus,
in deren DNS die HAV-Sequenzen /LIVP-pSPVV-HAV-D/ enthalten sind, gliedert man in den Rat2-Zellen im Medium
von Dulbecco's MEM mit einem 5%igen Serum der neugeborenen Kälber an. Das zu synthetisierende HAV-Protein in Form von Zellenlysate verwendet man für die Immunisierung von Meeresschweinchen und Kaninchen.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Die Mehrheit der immunisierten Meerschweinchen und Kaninchen enthielten Antikörper in ausreichend hohen Konzentration, die fähig waren, sich mit nativem Virus zu verknüpfen.

Beispiel 9. Rekombination-Plasmid-DNS pSPVV-HAV--D-protea.

5 μg Plasmids pSPVV-HAV-D, das ein Fragment des HAV-Genoms mit einer Länge von 3372 Basenpaaren aufweist, spaltet man teilweise mit PstI analog dem Beispiel 1. Die hergestellte lineare Form des Plasmids ligiert man mit PstI-Fragment des HAV-Genoms mit einer Länge von 924 Basenpaaren, das aus dem Plasmid pHAV 5-prot ausgeschieden wurde, das die virale Protease kodiert.

Mit dem Ligasegemisch transformiert man kompetente Zellen des Stammes E.coli DHI und in den Klonen, die Rekombination-Plasmid-DNS mit einem eingebauten Fragment des HAV-Genoms /pSPVV-HAV-D-protea, Fig. 12/ aufweisen, ermittelt man die Orientierung des letzteren mit Hilfe der Restriktionsendonukleasen der BamHI, PvuII, HindIII.

In der DNS - Plasmide, die Fragmente mit einer erforderlichen Orientierung enthalten, ermittelt man die Struktur der Angliederungen im Maxam-Gilbert-Verfahren.

Die DNS-Plasmide pSPVV-HAV-D-protea mit bestätigter Struktur der Angliederung kodieren die Synthese des Polypeptids, das alle strukturellen /beginnend mit 38 Aminosäuren des Proteins VP4/ und einige nichtstrukturelle HAV-Proteine aufweist. Diese Plasmide verwendet man für die Transformation von Nierenzellen der grünen Meerkatze der Linie CV1, die mit dem Pockenvakzine-Virus des Stammes LIVP infiziert wurden. Die Viruskultur wird nach der Transformation auf embryonale Fibroblaste einer Ratte der überimpften Linie Rat2, TK-Phenotyps, geimpft. In Gegenwart des Bromdesoxyuridins kloniert man die Rekombinationsviren. Die Klone der Pockenvakzine, deren DNS die HAV-Sequenzen /LIVP-pSPVV-HAV-D-protea/ enthalten, gliedert man an Rat2-Zellen im Medium von Dulbecco's MEM mit einem 5%igen Serum der neugeborenen Kälber an. Das synthetisierbare HAV-Protein wird in Form von Zellenlysaten für die Immunisierung von Meerschweinchen und Kaninchen verwendet.

In einigen Wochen nach dem Abschluß der Immunisierung testet man das Serum der Meerschweinchen und Kaninchen auf Gehalt an Antikörpern, die fähig sind, sich mit nativem HAV in einer Konkurrenzreaktion mit humanen Antikörpern zu verknüpfen.

Die Mehrheit der immunisierten Meerschweinchen und Kaninchen enthielten Antikörper in ausreichend hohen Konzentrationen, die fähig sind, sich mit nativem Virus zu verknüpfen.

Beispiel 10. Rekombination-Plasmid-DNS pSPVV-HAV-D.

Alle Operationen gehen wie in Beispiel 8 vor sich , man führt aber anstelle der Immunisierung von Kaninchen mit einem Zellenlysat ihre Vakzination mit gereinigtem Rekombinationsvirus der Pockenvakzine mit einem Titer $5 \times 10^9$ Partikel/ml durch. An der Seite eines Kaninchens rasiert man eine Fläche von 5 cm$^2$ und skarifiziert. Auf diese Oberfläche trägt man 0,1x0,2 ml Virus auf. Am 3. bis 4. Tag nach der Infizierung entsteht eine spezifische Entzündungsreaktion. Zum 20. Tag reinigt sich die Hautoberfläche. In 3 bis 4 Wochen nach der Infizierung wird das Serum entnommen. Sein Titer gegen die Pockenvakzine beträgt 1:640, 1:1280.

Das Serum weist auch Antikörper gegen HAV auf.

Industrielle Anwendbarkeit

Die erfindungsgemäßen Rekombination-Plasmid-DNS können für die Herstellung von Genengineering-Vakzine gegen Hepatitis A eingesetzt werden.

PATENTANSPRUCH

Rekombination-Plasmid-DNS, die die Synthese von Polypeptidsubstanzen für Vakzine gegen die Hepatitis A kodieren, die aus einem Fragment des HAV-Genoms und eines Expressionsvektors bestehen, wobei man als Fragment des HAV-Genoms verwendet: ein Fragment mit einer Länge von 389 Basenpaaren, das ein Fragment des VP1-Proteins mit 4 bis 129 Aminosäuren kodiert, ein Fragment mit einer Länge von 3372 Basenpaaren, das alle strukturellen, beginnend mit 38 Aminosäuren des VP4-Proteins, und nichtstrukturellen HAV-Proteine kodiert, ein Fragment mit einer Länge von 1243 Basenpaaren, das eine Polypeptidkette kodiert, die Fragmente der Proteine VP3 mit 110 bis 233 Aminosäuren und VP1 mit 1 bis 271 Aminosäuren aufweist, Fragmente mit einer Länge von 144 Basenpaaren, das ein Fragment des VP1-Proteins mit 4 bis 57 Aminosäuren kodiert, synthetisches DNS-Fragment, das ein Fragment des Proteins VP1 mit 11 bis 25 Aminosäuren kodiert, das folgende Struktur aufweist:

AATTCACAGTTAGTACTGAACAAAATGTACCAGATCCACAGGTAGGTATTGCG
    GTGTCAATCATGACTTGTTTTACATGGTCTAGGTGTCCATCCATA

oder ein Fragment mit einer Länge von 4296 Basenpaaren, das aus einem Fragment mit einer Länge von 3372 Basenpaaren besteht, das alle strukturellen und einige nichtstrukturelle HAV-Proteine kodiert, und das aus einem Fragment mit einer Länge von 924 Basenpaaren besteht, das Virusprotease kodiert, und als Expressionsvektor verwendet man Plasmide pUR 291, pUR 292, pIFN-fu 1, pIFN-fu 2 oder Plasmid pSPVV, das in den Vektor der Pockenvakzine LIVP eingebaut ist.

FIG. 1

ФАЙЛ=HAS-15    ОТ  1    ДО  3372

```
   1 GCAGTGACTG GAGCTTCTTA TTTCACTTCT GTGGACCAAT CTTCAGTTCA    50
  51 TACTGCTGAG GTTGGCTCAC ACCAAATTGA ACCTTGAAA ACCTCTGTTG   100
 101 ATAAACCTGG TTCTAAGAAA ACTCAGGGGG AGAAGTTTTT CTTGATTCAT   150
 151 TCTGCTGATT GGCTCACTAC ACATGCTCTC TTTCATGAAG TTGCAAAATT   200
 201 GGATGTGGTG AAACTGCTGT ACAATGAGCA GTTTGCCGTC CAAGGTTTGT   250
 251 TGAGATACCA TACTTATGCA AGATTGGCA TTGAGATTCA AGTTCAGATA   300
 301 AATCCCACAC CCTTTCAGCA AGGAGGACTA ATCTGTGCCA TGGTTCCTGG   350
 351 TGACCAAAGT TATGGTTCAA TAGCATCCTT GACTGTTTAT CCTCATGGTC   400
 401 TGTTAAATTG CAATATCAAC AATGTAGTTA GAATAAAGGT TCCATTTATT   450
 451 TATACTAGAG GTGCTTATCA TTTTAAAGAT CCACAGTACC CAGTTTGGGA   500
 501 ATTGACAATC AGAGTTTGGT CAGAGTTGAA TATTGGAACA GGAACCTCAG   550
 551 CTTATACTTC ACTCAATGTT TTAGCTAGGT TTACAGATTT GGAGTTGCAT   600
 601 GGATTAACTC CTCTTTCTAC ACAGATGATG AGAAATGAAT TTAGAGTTAG   650
 651 TACTACTGAA AATGTTGTAA ATTTGTCAAA TTATGAAGAT GCAAGGGCAA   700
 701 AAATGTCTTT TGCTTTGGAT CAGGAAGATT GGAAGTCTGA TCCTTCCCAA   750
 751 GGTGGTGGAA TTAAAATTAC TCATTTCACT ACCTGGACAT CCATTCCAAC   800
 801 CTTAGCTGCT CAGTTCCAT TTAATGCTTC AGATTCAGTT GGGCAACAAA   850
 851 TTAAAGTTAT ACCAGTGGAC CCATACTTTT TCCAGATGAC AAACACTAAT   900
 901 CCTGATCAAA AATGTATAAC AGCCTTGGCC TCTATTTGTC AGATGTTCTG   950
 951 CTTTTGGAGG GGAGATCTTG TTTTCGATTT TCAGGTTTTT CCAACCAAAT  1000
1001 ATCATTCAGG TAGGCTGTTG TTTTGTTTTG TTCCTGGGAA TGAGTTAATA  1050
1051 GATGTTACTG GAATTACATT AAAACAGGCA ACTACTGCTC CTTGTGCAGT  1100
1101 GATGGACATT ACAGGAGTGC AGTCAACCTT GAGATTTCGT GTTCCTTGGA  1150
1151 TTTCTGATAC ACCCTATCGA GTGAATAGGT ACACGAAGTC AGCACATCAA  1200
1201 AAAGGTGAGT ATACTGCCAT TGGGAAGCTT ATTGTGTATT GTTATAATAG  1250
1251 ATTGACTTCT CCTTCTAATG TTGCTTCTCA TGTTAGAGTT AATGTTTATC  1300
1301 TTTCAGCAAT TAATTTGGAA TGTTTTGCTC CTCTTTACCA TGCTATGGAT  1350
1351 GTTACCACAC AGGTTGGAGA TGATTCAGGA GGTTTCTCAA CAACAGTTTC  1400
1401 TACAGAGCAG AATGTTCCTG ATCCCCAAGT TGGCATAAAA GGGAAAGCCA  1450
1451 ATAGGGAAA GATGGATGTA TCAGGAGTGC AGGCACCTGT GGGAGCTATT  1500
1501 ACAACAATTG AGGATCCAGT TTTAGCAAAG AAAGTACCTG AGACATTTCC  1550
1551 TGAATTGAAG CCTGGAGAAT CCAGACATAC ATCAGATCAC ATGTCTATTT  1600
1601 ATAAATTCAT GGGAAGGTCT CATTTCTTGT GTACTTTTAC TTTTAATTCA  1650
1651 AACAATAAAG AGTACACATT TCCAATAACT CTGTCTTCGA CTTCTAATCC  1700
1701 TCCTCATGGT TTACCATCAA CATTAAGGTG GTTCTTTAAT TTGTTTCAGT  1750
1751 TGTATAGAGG ACCATTGGAT TTGACAATTA TAATCACAGG AGCCACTGAT  1800
1801 GTGGATGGTA TGGCCTGGTT TACTCCAGTG GGCCTTGCTG TCGACACCCC  1850
1851 TTGGGTGGAA AAGAAGTCAG CTTTGTCTAT TGATTATAAA ACTGCCCTTG  1900
1901 GAGCTGTTAG ATTTAATACA AGAGAACAG GGAACATTCA GATTAGATTG  1950
1951 CCATGGTATT CTTATTTGTA TGCCGTGTCT GGAGCACTGG ATGGCTTGGG  2000
2001 AGATAAGACA GATTCTACAT TTGGATTGGT TTCTATTCAG ATTGCAAATT  2050
2051 ACAATCATTC TGATGAATAT TTGTCCTTTA GTTGTTATTT GTCTGTCACA  2100
2101 GAGCAATCAG AGTTCTATTT CCCTAGAGCT CCATTAAATT CAAATGCTAT  2150
2151 GTTGTCCACT GAGTCCATGA TGAGTAGAAT TGCAGCTGGA GACTTGGAGT  2200
2201 CATCAGTGGA TGATCCCAGA TCAGAGGAGG ACAGAAGATT TGAGAGTCAT  2250
2251 ATAGAATGTA GGAAACCATA TAAAGAATTG AGACTGGAGG TTGGGAAACA  2300
2301 AAGACTCAAA TATGCTCAGG AAGAGTTATC AAATGAAGTG CTTCCACCTC  2350
2351 CTAGGAAAT GAAGGGGTTA TTTTCACAAG CTAAAATTTC TCTTTTTTAT  2400
2401 ACAGAGGAGC ATGAAATAAT GAAATTTTCT TGGAGAGGAG TGACTGCTGA  2450
2451 TACTAGGGCT TTGAGAAGAT TTGGATTCTC TCTGGCTGCT GGTAGAAGTG  2500
2501 TGTGGACTCT TGAAATGGAT GCTGGAGTTC TTACTGGGA ATTGATCAGA  2550
2551 TTGAATGATG AGAAATGGAC AGAAATGAAG GATGATAAGA TTGTTTCATT  2600
2601 AATTGAAAAG TTCACAAGCA ATAAATATTG GTCTAAAGTG AATTTTCCAC  2650
2651 ATGGAATGTT GGATCTTGAG GAAATTGCTG CCAACTCTAA AGATTTTCCA  2700
2701 AATATGTCTG AGACAGATTT GTGTTCCTG TTGCATTGGC TAAATCCAAA  2750
2751 GAAATTAAT TTAGCAGATA GAATGCTTGG ATTGTCTGGA GTGCAGGAA  2800
2801 TTAAAGAACA GGGTGTTGGA CTGATAGCAG AGTGTAGAAC TTTCTTGGAT  2850
2851 TCTATTGCTG GGACTTTGAA ATCTATGATT TTTGGGTTTC ATCATTCTGT  2900
2901 GACTGTTGAA ATTATAAATA TTGTGCTTTG TTTTATTAAG AGTGGAATCC  2950
2951 TGCTTTATGT CATACAACAA TTGAACCAAG ATGAACACTC TCACATAATT  3000
3001 GGTTTGTTGA GAGTTATGAA TTATGCAGAT ATTGGCTGTT CAGTTATTTC  3050
3051 ATGTGGTAAA GTTTTTTCCA AAATGTTAGA AACAGTTTTT AATTGGCAAA  3100
3101 TGGACTCTAG AATGATGGAG CTGAGGACTC AGAGCTTCTC CAATTGGTTA  3150
3151 AGAGATATTT GTTCGGGAAT TACTATTCTT AAAAGTTTTA AGGATGCCAT  3200
3201 ATATTGGTTA TGTACAAAAT TGAAGGATTT TTATGAAGTA AATTATGGCA  3250
3251 AGAAAAAGGA TGTTCTTAAT ATTCTCAAAG ATAACCAGCA AAAAATAGAA  3300
3301 AAAGCCATTG AAGAAGCAGA CAATTTTTGC ATTTTGCAAA TTCAAGATGT  3350
3351 GGAGAAATTT GATCAGTATC AG                                 3400
```

FIG.2

3

HAS-15-protea

AGTTGGCATTCTTGGAGTGCTTGTGGGAGGATGGTTTGTGTATAAGCATTTTTCCCGCA
AAGAGGAAGAACCAATTCCAGCTGAAGGGGTTTATCATGGCCGTGACTAAGCCCAAACAAGT
GATTAAATTGGATGCAGATCCAGTAGAGTCCCAGTCAACTCTACAAATAGCAGGATTAGTT
AGGAAAAATCTGGTTCAGTTTGCAGTTGGTGAGAAAAATGGATGTGTGAGATGGGTCATGA
ATGCCTTAGGAGTGAAGGATGATTGGTTGTTAGTACCTTCTCATGCTTATAAATTTGAAAA
GGATTATGAAATGATGGAGTTTTACTTCAATAGAGGTGGAACTTACTATTCAATTTCAGCT
GGTAATGTTGTTATTCAATCTTTAGATGTGGGATTTCAAGATGTTGTTTTAATGAAGGTTC
CTACAATTCCCAAGTTTAGAGATATTACTCAACACTTTATTAAGAAAGGAGATGTGCCTAG
AGCCTTAAATCGCTTGGCAACATTAGTGACAACCGTTAATGCAACTCCTATGTTAATTTCT
GAGGGACCATTAAAGATGGAAGAAAAAGCCACTTATGTTCATAAGAAGAATGATGGTACTA
CAGTTGATTTGACTGTAGATCAGGCATGGAGAGGAAAAGGTGAAGGTCTTCCTGGAATGTG
TGGTGGGGCCCTAGTGTCATCAAATGAGTCCATACAGAATGCAATTTTGGGTATTCATGTT
GCTGGAGGAAATTCAATTCTTGTGGCAAAGCTGGTTACTCAAGAAATGTTTCAAAACATTG
ATAAGAAAATTGAAAGTCAGAGAATAATGAAAGTGGAATTTACTCAATGTTCAATGAATGT
AGTCTCCAAAACGCTTTTTAGAAAGAGTCCCATTAATCACCACATTGATAAAACCATGATT
AATTTACCTGCA

FIG.3

4

‹‐galactosidase ──────►|‹──────── linker ────────►|‹VP1──►

...TGG TGT CGG GGA TCC GTC GAC CTG CAG GAT...
                     Bam HI             Pst I

*Pst I*

*Bam HI*

*Eco RI*

*Pst I*

*Hind III*

*Eco RI*

$Ap^R$

HAV22(VP1)
389 b.p.

Bam HI

pUR 291 - HAV22

Lac Z

po

FIG. 4

5

~galactosidase ∗ —————linker————— ∗VP4→

...TGG TGT CAG GGG ATC CGT CGA CCT GCA | GTG ...

BamHI     PstI

EcoRI
HindⅢ
PstI

PvuⅡ   BamHI
HindⅢ
BglⅡ

HAV23 3372 b.p.

PstI
EcoRI
BamHI

ApR

PUR 292 - HAV23

lac Z

po

FIG. 5

6

... AGG | AGT CAG ... TCC CAA | GAT CTG CAG GAT...

FIG. 6

FIG.7

FIG. 8

FIG.9

ს

FIG.10

IFN ←———— linker ———————→ *×* ←VP1→
                                        11  12  13
... CTG CAG GGT TCC GAA TTC ACA GTT AGT...
    Pst I           Eco RI

FIG.12

FIG.11

0276330

II

pSP-VV-HAV-D-protea
tk gene

Ori

Amp^r

tk gene

protease

promotor p7.5

HAV

BamHI

PstI

PstI

BamHI

PstI

synthetic linker

5'                    3'
GAT CCT ATG AGG ACT GCA
GA TAC TCC TG

FIG.12

Ori

Amp^r

pSP-VV-HAV-D

tk gene

tk gene

HAV

promotor p.7.5

BamHI

BamHI

PstI

PstI

synthetic polylinker

5'                    3'
GAT CCT ATG AGG ACT GCA
CA TAC TCC TG

FIG.11

**I. CLASSIFICATION OF SUBJECT MATTER** (if several classification symbols apply, indicate all) *

According to International Patent Classification (IPC) or to both National Classification and IPC

IPC$^4$    -C12 N15/00, A 61 K 39/29

**II. FIELDS SEARCHED**

| Minimum Documentation Searched [7] | |
|---|---|
| Classification System | Classification Symbols |
| IPC$^4$ | C 12 N 15/00, A 61 K 39/29 |

Documentation Searched other than Minimum Documentation
to the Extent that such Documents are Included in the Fields Searched [8]

| | |
|---|---|
| | |

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Doclady Akademii nauk SSSR, vol, 285, Nr.4, 1985 (Nauka, Moscow), Ju.A.Ovchinnikov et al "Posledovatelnost 3372 nukleotidnykh zveniev RNK virusa gepatita A, kodirujuschaya kapsidnye VP4-VP1 i nekotorye nestrukturnye belki", see pages 1014-1018, referred to in the description. --- | I |
| A | Proceedings of the National Academy of Sciences, vol.82, Nr.9, May 1985 (USA), Richard Najarian et al "Primary structure and gene organization of human hepatitis A virus", see pages 2627-2631, referred to in the description. --- | 1 |
| | ../.. | |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| 23 October 1987 (23.10.87) | 18 November 1987 (18.11.87) |
| International Searching Authority | Signature of Authorized Officer |
| ISA/SU | |

Form PCT/ISA/210 (second sheet) (January 1985)

| FURTHER INFORMATION CONTINUED FROM THE SECOND SHEET | | |
|---|---|---|
| A | Journal of Virology, vol.5 4,Nr.2, May 1985 (American Society for Microbiology), David L.Linemeyer et al."Molecular Coloning and Pertial Sequencing of-Hepatitis A Viral cDNA",see pages 247-255 | 1 |
| | --- | |
| A | EP,A2, 0061740, (Winnacker, Ernst-L. et al), 06 October 1982 (06.10.82), see pages 1,13, 14 | 1 |
| | --- | |
| A | EP,A2,0154587,(MERCK & CO.INC.), 11 September 1985 (11.09.85),see pages 1,27-30, referred to in the description. | 1 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers ............ because they relate to subject matter not required to be searched by this Authority, namely:

2.☐ Claim numbers ............ because they relate to parts of the international application that do not comply with the prescribed requirements to such an extent that no meaningful international search can be carried out, specifically:

3.☐ Claim numbers ........... because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)